# EUROPÄISCHE PATENTANMELDUNG

(11) **EP 4 775 391 A1**
(43) Veröffentlichungstag der Anmeldung: **15.07.2026**
(21) Anmeldenummer: 25150947.7
(22) Anmeldetag: 09.01.2025
(51) Int. Cl.: B32B 5/02, A61B 5/00, A61B 6/04, A61G 13/12, B32B 5/12, B32B 5/26

(54) **COMPOSITE-SCHALE**

(71) Anmelder: Siemens Healthineers AG, 91301 Forchheim (DE)
(72) Erfinder: Seidel, Christian, 91083 Baiersdorf (DE); Ntourmas, Felix, 96450 Coburg (DE); Seifert, Martin, 95447 Bayreuth (DE)
(74) Vertreter: Siemens Healthineers Patent Attorneys

(57) **Zusammenfassung**

Die Erfindung betrifft eine Composite-Schale wie sie z.B. Teil einer Patientenliege ist, die in der technischen Ausstattung in medizinischen Großgeräten allgemein vorkommt und/oder eine Composite-Schale als Teil eines biegebelastbaren Composite-Bauteils wie es bei Sitzen, Liegen, Ablagen und/oder bei der Innenausstattung von Fahrzeugen Anwendung findet. Durch die Erfindung wird erstmals Nachhaltigkeit bei der Herstellung von medizinischen Großgeräten, die Composite-Schalen der Liegebretter von Patientenliegen umfassen, technisch verwirklicht. Es ist demnach möglich - durch gezielten Einsatz von Vliesstoffen anstelle der teuren Endlosfasern in Composite-Schalen, z.B. bei Liegebrettern von Patientenliegen, Autositzen, sonstigen biegebelasteten Composite-Bauteilen - mechanisch nahezu gleich gute Bauteile mit im Kreislauf geführten Verstärkungsvliesstoffen aus recycelten und/oder Abfall-Fasern, anstatt mit neu produzierten Endlosfasern zu bauen.

## Beschreibung

### Technisches Gebiet

Die Erfindung betrifft eine Composite-Schale wie sie z.B. Teil einer Patientenliege ist, die in der technischen Ausstattung in medizinischen Großgeräten allgemein vorkommt und/oder eine Composite-Schale als Teil eines biegebelastbaren Composite-Bauteils wie es bei Sitzen, Liegen, Ablagen und/oder bei der Innenausstattung von Fahrzeugen Anwendung findet.

Eine Patientenliege liefert eine ergonomische, komfortable und funktionale Lösung für die Lagerung und/oder Behandlung eines Patienten im medizinischen Umfeld. Es gibt es Patientenliegen, die als Liegebrett eine derartige Composite-Schale als biegebelastbares Composite-Bauteil umfassen.

Beispielsweise werden in medizinischen Großgeräten wie Computertomographie (CT), in der molekularen Bildgebung (MI), bei Röntgengeräten und Magnetresonanztomographie (MRT) sowie dem Angiographie-Umfeld (AT) Patientenliegen zur Aufnahme und/oder Positionierung eines Patienten und dessen Bewegung durch die Scanebene eingesetzt. Daneben werden Patientenliegen auch bei einer radiotherapeutischen Bestrahlung mittels Röntgenstrahlung oder Partikelstrahlung, einer entsprechenden Bestrahlungsplanung oder einer medizinischen Intervention unter Bildgebung eingesetzt.

Die Auslegung von Patientenliegen für medizinische Anwendungen berücksichtigt immer zum einen eine mechanische Stabilität und/oder Steifigkeit der Composite-Schale und zum anderen die Durchlässigkeit und/oder das Abbildungs- und/oder Röntgenabsorptionsverhalten der Composite-Schale, welches sich auf die Bildqualität sowie die dem Patienten applizierte Röntgendosis auswirken kann. Composite-Schalen für Patientenliegen sind daher bevorzugt röntgentransparent ausgebildet.

Dabei besteht einerseits die Anforderung an die Patientenliege, möglichst leicht und dünnwandig zu sein, um die Strahlenbelastung und/oder Dosisleistung für den Patienten möglichst gering zu halten. Andererseits sind mechanische Vorgaben in Bezug auf Festigkeit und Steifigkeit zu erfüllen, da beispielsweise in der Anwendung auch Patienten mit hohem Gewicht - wie beispielsweise über 125 kg - bei hohen Auskragungen des Liegebretts - wie z.B. bei 1,5 m Hebelarm - durch die Scanebene des Gerätes bewegt werden. Dabei darf die Composite-Schale der Patientenliege nicht nur nicht brechen, sondern darf auch ein gewisses Toleranzmaß an Durchbiegung im Bereich der Scanebene nicht überschreiten, um den Patienten im Iso-Zentrum zu halten. Zu große Durchbiegungen ziehen möglicherweise Beeinträchtigungen in der Bildqualität und somit Beeinträchtigungen in der Diagnose nach sich.

Eine hohe mechanische Stabilität wird typischerweise durch erhöhten Materialeinsatz erreicht, was -beispielsweise - die Röntgentransparenz senkt. Die beschriebenen Auslegungskriterien Biegefestigkeit einerseits und Leichtigkeit respektive Transparenz gegenüber der angewendeten Strahlung andererseits sind also im Regelfall widerstreitend.

### Technischer Hintergrund

Patientenliegen nach dem Stand der Technik haben Composite-Schalen typischerweise aus Faser-Kunststoff-Composites und/oder Verbundmaterialien, beispielsweise mit einem Hartschaumkern. Ein Faserverbundwerkstoff ist ein im Allgemeinen zwei Hauptkomponenten - eine bettende Matrix und verstärkende Fasern - umfassender Mehrphasen- oder Mischwerkstoff. Dabei übernehmen die verstärkenden Fasern und die bettende Matrix sehr spezifische Aufgaben. Durch gegenseitige Wechselwirkungen der beiden Komponenten erhält der Faserverbundwerkstoff höherwertige Eigenschaften als jeder der beiden einzeln beteiligten Komponenten. Das bedingt aber auch, dass die beiden beteiligten Materialien sehr unterschiedliche Eigenschaften haben.

Es kommen beispielsweise Endlosfaser-verstärkte Kunststoffe auf Basis von Kohlefasern (CFK) zum Einsatz. Beispielsweise bilden Duroplaste und/oder Thermoplaste, sowie beliebige Gemische und Blends daraus eine bettende Matrix. Beispielsweise umfassen die Composite-Schalen für Patientenliegen Composite-Werkstoffe, die im Querschnitt Hybridaufbauten aus CFK-Lagen mit unterschiedlichen Faserorientierungen zeigen, wobei insbesondere die Biegehauptbelastung bauteillängs orientiert ist. Dabei ist beispielsweise ein Schichtaufbau einer Composite-Schale realisiert und es können 100 oder mehr Einzellagen mit verschiedener, zum Teil gegensätzlicher Faserorientierung vorliegen.

Es gibt bei Composite-Schalen den so genannten Sandwichaufbau, wobei zwei biegebelastbare Composite-Schichtstapel einen Kern aus einem Hartschaumstoff und/oder aus sonstigen Kernwerkstoffen umschließen. Diese Sandwichlösungen lassen sich in der Regel durch einen Pressverarbeitungsprozess von Nasslaminat über Hydraulische Pressen und/oder Autoklav mit Vakuumsackaufbau herstellen. Ein bekannter Sandwich-Aufbau einer Patientenliege ist beispielsweise in der DE 102022205106 beschrieben.

Weiterhin gibt es den monolithischen Aufbau, wobei die Bauteile / Patientenliegen auch im Querschnitt nur aus Faserlaminat bestehen. Diese werden beispielsweise durch Flüssigharzinjektionsverfahren von den trockenen Verstärkungsfasern produziert.

Bisher gibt es keinen Ansatz, das Material einer Composite-Schale egal welchen Aufbaus, ob monolithisch oder Sandwich-Struktur, bei Patientenliegen in einen Materialstoff-Kreislauf einzubringen. Es besteht daher der Bedarf an technischen Lösungen, bei denen die Materialtechnik der Composite-Schalen, insbesondere von Patientenliegen, von einem Material-Kreislauf, zumindest teilweise, gespeist wird.

Aufgabe der vorliegenden Erfindung ist es, eine nachhaltige Materialtechnik auf Basis recycelter Verstärkungsfasern und/oder auf Basis von Abfall-Fasern für eine Composite-Schale z.B. als Teil einer Patientenliege, bei vergleichbaren technischen Eigenschaften zu den Composite-Schalen aus ausschließlich neuen Fasern, zur Verfügung zu stellen.

### Zusammenfassung der Erfindung

Diese Aufgabe wird durch den Gegenstand der vorliegenden Erfindung, wie er in der Beschreibung, den Ansprüchen und den Figuren offenbart ist, gelöst.

Dementsprechend ist Gegenstand der vorliegenden Erfindung eine Composite-Schale im Sandwich-Aufbau, zwei äußere Composite-Schichtpakete als äußere Lagen und mittig einen Kern umfassend, wobei im Kern und/oder in den beiden umgebenden äußeren Composite-Schichtpaketen Vliesstoff-verstärktes Composite-Material, das im Vliesstoff Fasern aus Recycling und/oder Produktionsausschuss umfasst, enthalten ist.

### Ausgestaltungen der Erfindung

Nach einer Ausführungsform ist die Composite-Schale ein Teil eines Liegebretts einer Patientenliege.

Nach einer weiteren Ausführungsform umfasst der Kern Vliesstoff-verstärktes Composite-Material.

Nach einer Ausführungsform umfasst der Kern der Composite-Schale ein Composite-Material mit Vliesstoff-Verstärkung in Form von Bulkmaterial.

Nach einer weiteren Ausführungsform umfasst ein Composite-Schichtpaket bis zu 300, insbesondere bis zu 150 Einzellagen.

Nach einer Ausführungsform ist die Composite-Schale symmetrisch um den Kern herum aufgebaut, so dass die beiden äußeren Lagen in Form von Composite-Schichtstapel hinsichtlich der Anzahl der Einzellagen gleich viele Einzellagen aufweisen.

Nach einer Ausführungsform ist die Composite-Schale symmetrisch um den Kern herum aufgebaut, so dass die beiden Composite-Schichtstapel gleiche und/oder gespiegelte, also achsensymmetrische Einzellagen-Anordnung hinsichtlich der Orientierung der Einzellagen mit Endlos-Faserverstärkung haben.

Nach einer Ausführungsform umfasst zumindest eine Einzellage eines Composite-Schichtpakets eine Faserverstärkung mit orientierten Endlosfasern.

Nach einer Ausführungsform umfasst zumindest eine Einzellage eines Composite-Schichtpakets Vliesstoff-Verstärkung.

Nach einer Ausführungsform liegt in zumindest einem Composite-Schichtstapel ein Lagenaufbau vor, bei dem die Faserorientierung zweier aufeinanderfolgender Einzellagen mit Faserverstärkung aus orientierten Endlosfasern um jeweils 90° gegeneinander verdreht sind.

Nach einer Ausführungsform liegt in einer Composite-Schale ein Sandwich-Aufbau mit folgender Einzellagen-Abfolge vor,
- 2 oder ein Vielfaches von 2 - Einzellagen mit Faserverstärkung und zumindest zum Teil unterschiedlich orientierten Endlosfasern,
- Kern aus Bulk-Composite-Material mit Vliesstoff-Verstärkung,
- 2 oder ein Vielfaches von 2 Einzellagen mit Faserverstärkung und zumindest zum Teil unterschiedlich orientierten Endlosfasern,

Nach einer weiteren Ausführungsform liegt in einer Composite-Schale ein Sandwich-Aufbau mit folgender Einzellagen-Abfolge vor,
- 2 oder ein Vielfaches von 2 - Einzellagen mit Faserverstärkung, wobei zumindest zwei aneinander angrenzende Einzellagen um 90° gedreht orientierte Endlosfasern aufweisen,
- im Kern mehrere Einzellagen mit Vliesstoff-Verstärkung,
- 2 oder ein Vielfaches von 2 - Einzellagen mit Faserverstärkung, wobei zumindest zwei aneinander angrenzende Einzellagen um 90° gedreht orientierte Endlosfasern aufweisen.

Als "Sandwich-Struktur" wird vorliegend ein Aufbau bezeichnet, der eine Mehrschichtmateriallösung zeigt, wobei zwei äußere und ein mittlerer Bereich vorliegt und der mittlere Bereich auch als Kern bezeichnet wird. Im Grenzfall kann auch der bekannte monolithische Aufbau einer Composite-Schale hier unter den Begriff "Sandwich-Struktur" fallen, wenn der Kern und die umgebenden Composite-Schichtstapel gleich aufgebaut sind, also eine identische Mehrschichtmateriallösung aufweisen.

Der Kern im herkömmlichen Sandwich-Aufbau umfasst nach dem Stand der Technik überwiegend kompakte und/oder geschäumte Kunststofflösungen mit und ohne Faserlösungen.

Vorliegend wird im Gegensatz dazu vorgeschlagen, den Kern aus einer - als Bulk-Composite-Materialkern - und/oder mehreren Einzellagen mit Vliesstoff-Verstärkung zu machen, wobei der Kern, wie bereits erwähnt, sich nicht zwangsläufig im Composite-Material und Schichtstapel-Aufbau von den umgebenden äußeren Lagen unterscheidet.

Die Dicke oder Wandstärke der Composite-Schale liegt im Bereich von 0,5 bis 100mm, vorzugsweise 5 bis 50 mm und besonders bevorzugt im Bereich 7 bis 35 mm.

Die Dicke des Kerns liegt dabei im Bereich von 0,1 mm bis 50 mm, vorzugsweise 0,5 mm bis 30mm und besonders bevorzugt 0,7mm bis 20mm.

Die Dicke der äußeren Lagen in Form von Composite-Schichtstapel ist, je nach Innenaufbau im Bereich der Dicke des Kerns, gleich der Dicke des Kerns oder -beispielsweise - darunter, sie liegt entsprechend zwischen 0,1 bis 30mm, bevorzugt 0,5 bis 15 mm und besonders bevorzugt im Bereich 1 bis 10 mm.

Eine typische Dicke einer Einzellage ist ungefähr im Bereich 0,1 bis 1 mm.

Die Dicke eines Vliesstoff-verstärkten Bulk-Composite-Materialkerns als Formkörper liegt im Bereich von 0,1 bis 50mm.

Ein Composite-Schichtstapel bildet eine äußere Lage und umfasst eine Reihe von Einzellagen aus Composite-Material. Als "Composite-Material" wird ein Material aus Verstärkungsfasern in einer Kunststoffmatrix bezeichnet. Die Faserverstärkung bildet den "steifen" Teil des faserverstärkten Kunststoffes und liegt beispielsweise in Form von Fasern, Faserbündel, Rovings, Fasergelegen, klassischen Webstrukturen und/oder als Vliesstoff vor.

Die Vliesstoffe umfassen sowohl Vliesstoffe, die flächig vorliegen, in Gestalt einer Schicht, Lage, Folie und/oder Einzellage, z.B. so genannte Sheet-Materialien, als auch Vliesstoffe, die dreidimensionale und unregelmäßige Faser-Verbunde bilden, die vor der Verarbeitung mit Harz zu Composite-Material wie Wolken aussehen und die so genannte Bulk-Materialien sind.

Vorliegend wird als "Vliesstoff-verstärkt" ein Composite-Material bezeichnet, das recycelte und/oder Abfall-Fasern umfasst. Das Vliesstoff-verstärkte Composite-Material ist Teil eines Kunststoff-Kreislaufs.

Verstärkungsfasern können dementsprechend als Endlosfasern, als Faserbündel, als einzelne Fasern bestimmter Länge, als Faserverbunde, als Fasergelege, als Fasergewebe und/oder als Vliesstoffe vorliegen. Bei der kostspieligen textilen Primärfertigung teurer Endlosfasern, wie der Carbonfasern, beispielsweise der unidirektionalen "UD" Carbonfasern und/oder der hochmoduligen Carbonfasern, sowie der HT "HighTenacity" Carbonfasern, fallen so genannte "Verschnitt" Abfälle an, die in Form von Vliesstoffen einsetzbar sind. Diese werden vorliegend auch als "Abfall-Fasern" bezeichnet.

Ein Vliesstoff ist, im Gegensatz zur einfachen Faser, dem Gewebe, dem definierten Faserverbund und/oder der definierten Faser-Roving, eine ungeordnete statistische Anordnung "non-woven" von Fasern und/oder Faserbruchstücken. Der Nachweis des Einsatzes von Vliesstoff-Faserlösungen in faserverstärkten Kunststoffen erfolgt beispielsweise durch optische Auswertung, weil ein Vliesstoff eine andere Optik als ein Faser-Gewebe, Faser-Gelege und/oder Faserverbund hat. Definierte Anordnungen von Fasern sind in der Regel durch klassische Webstrukturen gekennzeichnet, wohingegen eine Vliesstoffstruktur willkürlich und ungeordnet ist. Im Englischen wird als Vliesstoff beispielsweise "nonwoven" bezeichnet, also alle Arten der Anordnung von Fasern außer einer regelmäßigen, wie sie beim Weben entsteht, ist unter dem Begriff "Vliesstoff" zu verstehen. Vliesstoffe sind flexible textile Flächengebilde oder dreidimensionale Bulkstoffe.

Typische Faserlängen im Vliesstoff liegen im Bereich einer Untergrenze von 2mm bis 7mm bis zu einer Obergrenze von 300mm bis 400mm. Beispielsweise liegen sie im Bereich 8mm bis 170mm, insbesondere im Bereich von 10mm bis 100mm.

Als Fasern für den Vliesstoff kommen alle gängigen anorganischen und/oder organischen Verstärkungsfasern, vorzugsweise recycelte Fasern und/oder Abfall-Fasern, sowie beliebige Kombinationen und Mischungen in Betracht. Beispielsweise sind das alle Arten von Kohlenstofffasern, Glasfasern, Organikfasern, wie z.B. Aramidfasern, PET(Polyethylenterephthalat)-Fasern, PP (Polypropylen)-Fasern, Cellulose, Keramikfasern- z.B. Metalloxide, wie z.B. Korundfaser Al₂O₃, Siliziumcarbidfasern. Die vorgenannten Fasern können einzeln oder im Gemisch sowie in beliebigen Kombinationen im Vliesstoff eingesetzt werden.

Beispielsweise können verschiedene Faserarten und/oder Faserqualitäten gebündelt in Rovings, respektive als Pultrudat, zusammengefasst zum Aufbau eines Vliesstoffes eingesetzt werden. Durch die Kombinationen verschiedener Verstärkungsfasern lassen sich alle Arten von physikalischen und chemischen Eigenschaften des resultierenden Vliesstoff-Faserverstärkten Composite-Materials gezielt einstellen und beeinflussen.

Als polymere Matrix wird beispielsweise ein duromeres Harz, basierend auf einer der Verbindungen z.B. Glycidylether, Novolake, Epoxidharze, Vinylesterharze, Polyurethane, Polyester, Silikone, Polyethylen, Ultrahochmolekulares Polyethylen, sowie beliebige Mischungen, Blends, Copolymere der vorgenannten Verbindungen, einsetzbar sind. wie oben beschrieben, eingesetzt. Des Weiteren können verschiedene Thermoplasten wie Polyamid, Polyethylenterephthalat, Polypropylen wiederum sowie beliebige Mischungen, Blends, Copolymere der vorgenannten Verbindungen, eingesetzt werden.

Vliesstoffe können flächige Composit-Materialien oder auch Bulk-Materialien durch Schichtung von Einzellagen ergeben. Beispielsweise können sheet moulding compounds "SMCs" oder bulk moulding compounds "BMCs" auch Vliesstoff-verstärkt unter Einsatz von recycelten Fasern hergestellt und/oder vorliegend zum Aufbau einer Composite-Schale eingesetzt werden.

Zur Herstellung der Vliesstoffe werden verschiedene Fasern, Faserreste, Recyclingfasern eingesetzt. Bekannt sind Recyclingfasern, also Fasern, die aus gebrauchten Materialien und/oder Produkten wiedergewonnen wurden. Diese Fasern liegen teilweise als Gewebe, als Maschenware, als Gestricke und/oder Gewirke vor. Zudem gibt es Abfallfasern, die zwar fabrikneu sind, allerdings aus Produktionsabfällen wie Verschnitt, Produktionsausschuss und dergleichen stammen.

Zur Herstellung des Composite-Materials wird beispielsweise die Faserverstärkung vorgelegt und mit flüssigem Harz getränkt, imprägniert und/oder in ein Bad mit Harz eingetaucht. Danach wird der dadurch gebildete Verbund durch Temperatur, Trocknung etc. gehärtet, wodurch sich der faserverstärkte Kunststoff, das Composite-Material, bildet. Die Polymerisation des zunächst flüssigen Matrixmaterials erfolgt je nach Harz mit Härter, als additive und/oder radikalische Polymerisation oder ohne Härter als Homopolymerisation mit einem Starter oder Initiator.

Zur Bildung des Composite-Schichtstapels werden mehrere dünne Einzellagen aus dem Composite-Material aufeinandergelegt, dann wird das so aufgebaute Lagenpaket beispielsweise in einen Foliensack eingepackt, unter Druck evakuiert, in einen Autoklaven verbracht und bei einem Autoklaven Druck von ca. 10 bar und erhöhter Temperatur thermogeformt.

### Exemplarische Ausführungsbeispiele der Zeichnung

- Figur 1: zeigt ein Designbeispiel für eine Composite-Schale nach einer beispielhaften Ausführungsform der Erfindung,
- Figur 2: zeigt einen beispielhaften Lagenaufbau einer Composite-Schale,
- Figur 3: zeigt für eine beispielhafte Ausführungsform der Erfindung den Einfluss der Recycling-Modifikation auf die Biegesteifigkeit einer Composite-Schale

### Detaillierte Beschreibung der Ausführungsbeispiele

### Erläuterung der Figuren

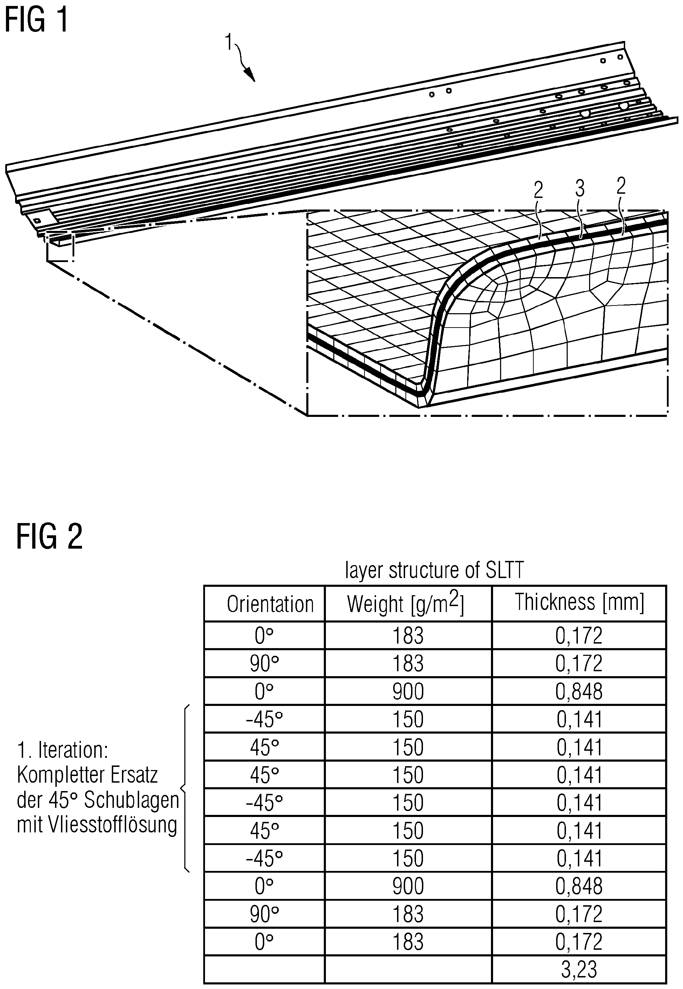

In Figur 1 ist eine Composite-Schale 1 zu erkennen. Die Composite-Schale 1 zeigt eine Schalenform, wie es bei Liegebrettern, beispielsweise für Patientenliegen, übliche Formgebung ist. Die Figur 1 zeigt außerdem ein Detail aus der Composite-Schale 1 in Vergrößerung, so dass die Sandwichstruktur erkennbar ist. Man erkennt die beiden äußeren Lagen in Form von Composite-Schichtstapel 2, die einen Kern 3 umgeben. Die Composite-Schichtstapel 2 umfassen jeweils eine Reihe von Einzellagen, die jeweils entweder Faserverstärkt oder Vliesstoff-verstärkt vorliegen. Der Kern 3 umfasst beispielsweise Vliesstoff-verstärktes Composite-Material entweder in Form von Einzellagen und/oder in Form eines Bulkmaterials.

Als "Faserverstärkt" wird vorliegend bezeichnet, wenn im Composite-Material eine Faserverstärkung mit Fasern, beispielsweise Endlosfasern, vorliegt, entsprechend wird als "Vliesstoff-verstärkt" das bezeichnet, wenn ein Vliesstoff - mit entsprechendem Anteil an recycelten Fasern - als steifer Bestandteil im Composite-Material vorliegt.

Figur 2 zeigt tabellarisch den Lagenaufbau einer Composite-Schale 2. Die Tabelle zeigt eine Faserverstärkung mit Orientierung der Verstärkungsfaser, so dass zur Einzellage die Daten der Orientierung [Angabe des Winkels °] der Endlosfaser in der Spalte ganz links, in der mittleren Spalte die Daten zum Gewicht der Einzellage in [g/m²] und ganz rechts die Daten zur Dicke der Lage in [mm] abzulesen sind. Die Orientierungen der Einzellagen mit Faserverstärkung sind demnach bei 0° in Längsrichtung, bei 90° in Querrichtung, bei +/- 45° -Richtung so angeordnet, dass sich in alle Richtungen ungefähr gleiche Eigenschaften ergeben und die Composite-Schale deshalb Längs-, Quer- und/oder Torsionsbelastungen gleich gut standhält.

In einem Test wurden nun sechs mittige Einzellagen aus der Composite-Schale durch Vliesstoff-verstärkte Einzellagen mit im Kreislauf geführtem Fasermaterial und/oder Verschnitt- und/oder Abfall-Fasermaterial ersetzt und die Biegesteifigkeit der beiden Composite-Schalen miteinander verglichen.

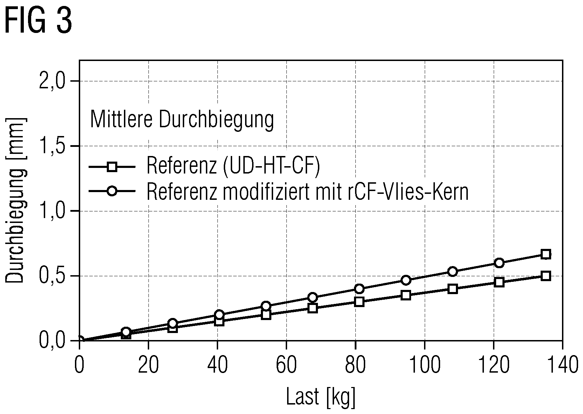

Figur 3 zeigt die Resultate der Tests wie oben beschrieben in graphischer Form. Aufgetragen wurde in der Abszisse die Last in [kg] und in der Ordinate die Durchbiegung in [mm]. Der untere Graph mit den Messpunkten in quadratischer Form zeigt eine herkömmlich, wie in der Tabelle von Figur 2, aufgebaute Composite-Schale mit ausschließlich Einzellagen aus orientierten Endlos-Einzelfaserverstärkung und ganz knapp darüber, also unwesentlich weniger biegesteif eine - nach einer beispielhaften Ausführungsform der Erfindung - mit Vliesstoff-verstärkten Einzellagen modifizierte Composite-Schale, deren Messwerte mit rund eingetragenen Graphik-Punkten dargestellt sind.

Zu erkennen ist klar, dass sich die Bauteilsteifigkeit einer Composite-Schale beim Austausch der teuren Endlosfaser in der Faserverstärkung durch Vliesstoff-Verstärkung aus recycelten und/oder Abfall-Fasern nur gering verschlechtert.

Hier konnte gezeigt werden, dass Nachhaltigkeit bei der Herstellung von Composite-Schalen möglich ist, weil die Vliesstoff-Verstärkung von Einzellagen - abhängig von dem Einbau der Einzellagen in die Composite-Schichtstapel der Sandwichstruktur - nahezu gleich gute Testergebnisse zur Biegesteifigkeit erbringt wie die reine Faserverstärkung. Damit ist eine nutzbare Kreislaufführung von recycelten Fasern, wie auch von Faserabfällen bei der Herstellung von Composite-Schalen technisch realisierbar.

Vorteile ergeben sich insbesondere bei der Ressourceneffizienz, beim Einsatz von Recyclingmaterial, beim ökologischen und ökonomischen Fußabdruck. Das Faserrecycling kann in diesem Zusammenhang mit einem stofflichen CO2-Fußabdruck von Null betrachtet werden. Damit werden die Nachhaltigkeitsverbesserungen für einen hochenergetischen Rohstoff in multiplen Aspekten ermöglicht und gefördert.

In der Anwendung werden die genannten Bauteile teilweise oder komplett aus "recycelten Carbonfasern" rCF-basierenden oder stofflich anderen Vliesstoffen ersetzt, beispielweise in der Querschnittsmitte im Bereich der neutralen Faser bei Biegebelastung.

Weiterer Vorteil für den genannten Ersatz der +/-45° Endlosfaserlagen ist ein Kostenvorteil im Falle der rCF-Materialien als Vliesstoffe, da die teure CF-Faserherstellung entfällt.

Durch die Erfindung wird erstmals Nachhaltigkeit bei der Herstellung von medizinischen Großgeräten, die Composite-Schalen der Liegebretter von Patientenliegen umfassen, technisch verwirklicht. Es ist demnach möglich - durch gezielten Einsatz von Vliesstoffen anstelle der teuren Endlosfasern in Composite-Schalen, z.B. bei Liegebrettern von Patientenliegen, Autositzen, sonstigen biegebelasteten Composite-Bauteilen - mechanisch nahezu gleich gute Bauteile mit im Kreislauf geführten Verstärkungsvliesstoffen aus recycelten und/oder Abfall-Fasern, anstatt mit neu produzierten Endlosfasern zu bauen.

Unabhängig vom grammatikalischen Geschlecht eines bestimmten Begriffes, wie z.B. "Patient" sind Personen mit männlicher, weiblicher oder anderer Geschlechteridentität mit umfasst.

## Patentansprüche

1. Composite-Schale im Sandwich-Aufbau, zwei äußere Composite-Schichtpakete als äußere Lagen und mittig einen Kern umfassend, wobei im Kern und/oder in den beiden umgebenden äußeren Composite-Schichtpaketen Vliesstoff-verstärktes Composite-Material, das im Vliesstoff Fasern aus Recycling und/oder Produktionsausschuss umfasst, enthalten ist.

2. Composite-Schale nach Anspruch 1, wobei der Kern recycelte Fasern in einer Vliesstoff-Verstärkung aufweist.

3. Composite-Schale nach Anspruch 1, wobei die Composite-Schale Teil einer Patientenliege für diagnostische und/oder therapeutische medizinische Geräte ist.

4. Composite-Schale nach einem der vorhergehenden Ansprüche 1 oder 2, wobei der Kern der Composite-Schale Composite-Material mit Vliesstoff-Verstärkung in Form von Bulkmaterial umfasst.

5. Composite-Schale nach einem der vorhergehenden Ansprüche, wobei in einem Composite-Schichtpaket bis zu 300 Einzellagen realisiert sind.

6. Composite-Schale nach einem der vorhergehenden Ansprüche, wobei die Composite-Schale symmetrisch um den Kern herum aufgebaut ist.

7. Composite-Schale nach einem der vorhergehenden Ansprüche, wobei die Composite-Schale zumindest eine Einzellage in einem Composite-Schichtpaket mit einer Faserverstärkung aus orientierten Endlosfasern aufweist.

8. Composite-Schale nach einem der vorhergehenden Ansprüche, die zumindest eine Einzellage in einem Composite-Schichtpaket mit einer Faserverstärkung aus Vliesstoff-Verstärkung aufweist.

9. Composite-Schale nach einem der vorhergehenden Ansprüche, wobei zumindest in einem Composite-Schichtstapel ein Lagenaufbau vorliegt, bei dem die Faserorientierung zweier aufeinanderfolgender Einzellagen mit Faserverstärkung aus orientierten Endlosfasern um jeweils 90° gegeneinander verdreht sind.

10. Composite-Schale nach einem der vorhergehenden Ansprüche, deren Wandstärke im Bereich 0,1 bis 100 mm liegt.

11. Composite-Schale nach einem der vorhergehenden Ansprüche, bei der ein Sandwich-Aufbau mit folgender Einzellagen-Abfolge vorliegt:
- 2 oder ein Vielfaches von 2 - Einzellagen mit Faserverstärkung und zumindest zum Teil unterschiedlich orientierten Endlosfasern,
- Kern aus Bulk-Composite-Material mit Vliesstoff-Verstärkung,
- 2 oder ein Vielfaches von 2 Einzellagen mit Faserverstärkung und zumindest zum Teil unterschiedlich orientierten Endlosfasern.

12. Composite-Schale nach einem der vorhergehenden Ansprüche, bei der ein Sandwich-Aufbau mit folgender Einzellagen-Abfolge vorliegt:
- 2 oder ein Vielfaches von 2 - Einzellagen mit Faserverstärkung, wobei zumindest zwei aneinander angrenzende Einzellagen um 90° gedreht orientierte Endlosfasern aufweisen,
- im Kern mehrere Einzellagen mit Vliesstoff-Verstärkung
- 2 oder ein Vielfaches von 2 - Einzellagen mit Faserverstärkung, wobei zumindest zwei aneinander angrenzende Einzellagen um 90° gedreht orientierte Endlosfasern aufweisen.

13. Composite-Schale nach einem der vorhergehenden Ansprüche, die ein Sheet-moulding-compound-Material "SMC" und/oder ein Bulk-moulding-compound-Material "BMC", das zumindest teilweise Vliesstoff aus recycelten Fasern aufweist, umfasst.
